(19) Europäisches Patentamt
European Patent Office
Office européen des brevets

(11) Publication number: **0 029 740 A1**

(12) # EUROPEAN PATENT APPLICATION

(21) Application number: 80304227.4

(22) Date of filing: 25.11.80

(51) Int. Cl.$^3$: C 07 D 333/22

(30) Priority: 27.11.79 FI 793710

(43) Date of publication of application: 03.06.81 Bulletin 81/22

(84) Designated Contracting States: AT BE CH DE FR GB IT LI LU NL SE

(71) Applicant: Farmos-Yhtymä Oy
P.O. Box 425
SF-20101 Turku 10(FI)

(72) Inventor: Koskenniska, Lasse Antero
Liistetie 4 A 2
SF-90650 Oulu 65(FI)

(72) Inventor: Saxlund, Raimo Antero
Koskitie 26 E 15-16
SF-95100 Oulu 50(FI)

(74) Representative: Collier, Jeremy Austin Grey et al,
J.A.Kemp & Co. 14, South Square Gray's Inn
London WC1R 5EU(GB)

(54) **Process for the preparation of a therapeutically active substituted phenoxyacetic acid and a novel acid anhydride for use therein.**

(57) Tienilic acid, [2,3-dichloro-4-(2-thienyl-carbonyl)-phenoxy] acetic acid, is advantageously made by reacting 2,3-dichloro-4-(2-thienylcarbonyl)-phenol with chloroacetic acid anhydride and hydrolysing the novel anhydride, *viz.* [2,3-dichloro- 4-(2-thienylcarbonyl)- phenoxy]acetic acid anhydride, so produced.

EP 0 029 740 A1

DESCRIPTION

"PROCESS FOR THE PREPARATION OF A THERAPEUTICALLY ACTIVE SUBSTITUTED PHENOXYACETIC ACID AND A NOVEL ACID ANHYDRIDE FOR USE THEREIN"

This invention relates to the preparation of [2,3-dichloro-4-(2-thienylcarbonyl)phenoxy]-acetic acid or tienilic acid of the formula:

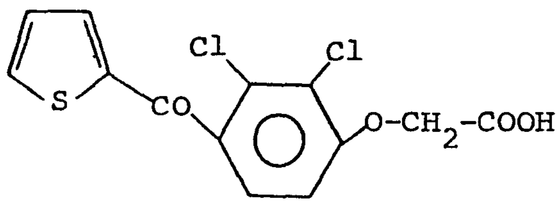

I

Tienilic acid is a known diuretic agent. The preparation of tienilic acid has been described in e.g. the French Patents Nos. 2,068,403 and 2,115,042.

According to French Patent No. 2,068,403 tienilic acid is prepared from the ketone of formula:

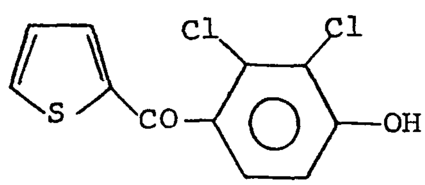

II

which is reacted in ethanolic solution with sodium methylate and chloroacetic acid ethyl ester, after which the tienilic acid ethyl ester formed is isolated and hydrolysed to give tienilic acid. The total yield is 68.7 per cent.

In the process of French Patent No. 2,115,042 the sodium salt of the ketone of formula II is reacted with the sodium salt of chloroacetic acid in aqueous solution. The yield of tienilic acid is 60 per cent.

It has now surprisingly been discovered that tienilic acid can be produced in a yield of 90-95 per cent if tienilic acid anhydride is made as an intermediate. The process of the present invention therefore comprises reacting 2,3-dichloro-4-(2-thienylcarbonyl)-phenol of formula (II) with chloroacetic acid anhydride in the presence of an alkaline condensation agent to give tienilic acid anhydride of formula (III) below, which is then hydrolysed to tienilic acid. The reaction can be represented by the following formulae:

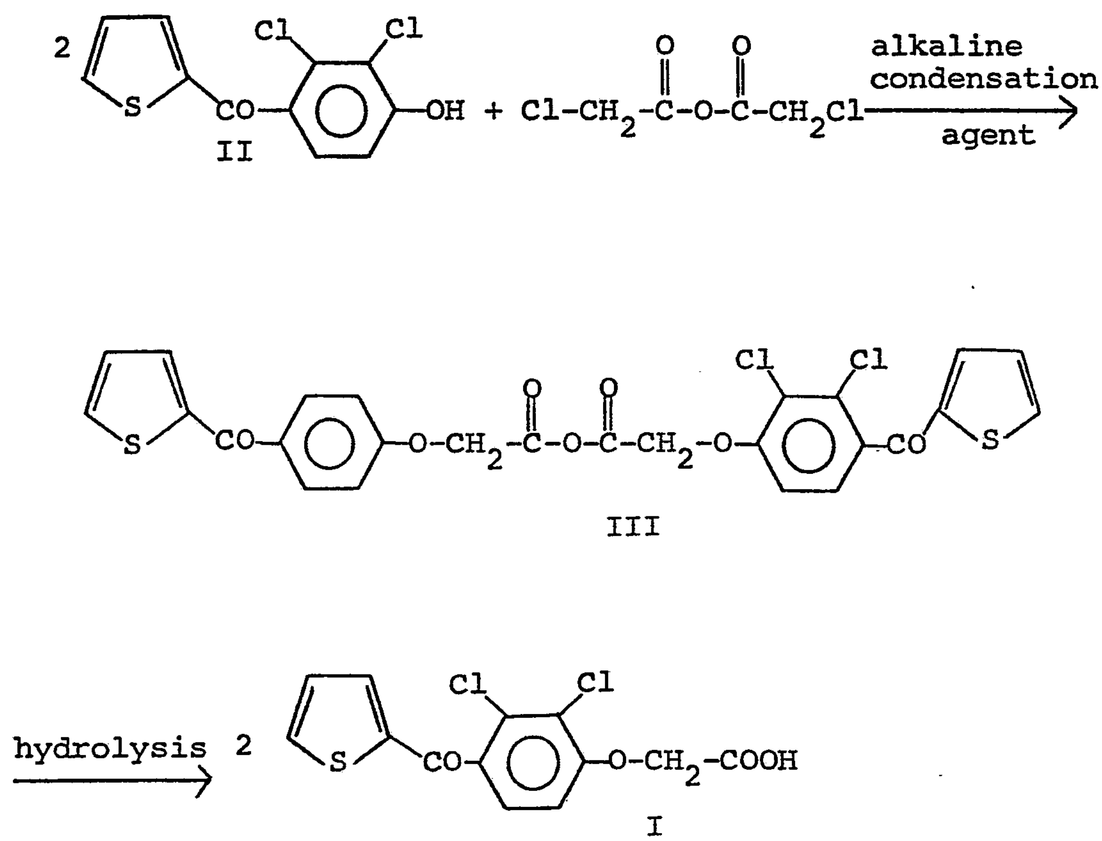


II

III

I

Tienilic acid anhydride is a new chemical compound. It is preferably prepared from the ketone of formula II and chloroacetic acid anhydride by refluxing in a suitable organic solvent, which may be for example, an aromatic or aliphatic hydrocarbon, a ketone, an ester, a halogenated hydrocarbon, an organic amide or a sulfoxide. The alkaline condensation agent used may be, e.g., sodium or potassium carbonate. The addition of a quaternary ammonium salt together with the alkali carbonate has proved to be especially favourable. A suitable ammonium salt is, e.g., Aliquat 336 (trioctylmethylammonium chloride). The addition of the ammonium salt accelerates the reaction considerably and increases the yield.

When the desired product is tienilic acid itself, it is not necessary to isolate the intermediate anhydride of formula III. The hydrolysis can be performed in the same solution as the condensation by conventional methods, for instance by adding sodium hydroxide solution and refluxing. By extraction of the cooled solution with a suitable solvent, e.g., hot toluene, and cooling the extract, pure tienilic acid is obtained.

In comparison with the known methods the process of the present invention gives much higher yields. Chloroacetic acid anhydride is a solid, almost odourless, reagent, which is easy to handle. The alkali metal carbonates and the ammonium salts are likewise easily handled. As the reaction, including the purification, can be performed in the same reaction vessel, the process of the invention is also simpler to perform than the known processes. It is thus economical and easily realizable on a technical scale.

The invention is illustrated by the following Examples:

Example 1

10 g of 2,3-dichloro-4-(2-thienylcarbonyl)-phenyl, 4.0 g of chloroacetic acid anhydride, 50 ml of toluene, 8.0 g of sodium carbonate and 4.0 ml of dimethylsulfoxide are refluxed for 14 hours. The mixture is then cooled, 50 ml of water and 5 ml of 48 per cent sodium hydroxide solution are added, and refluxing is continued for a further 2 hours. The phases are separated and the toluene layer washed with water (20 ml). The combined aqueous layers are acidified with hydrochloric acid (to pH = 2-3), and the product is extracted with hot toluene. The toluene solution is cooled and the product is filtered off. 11.0 g (90.0%) of almost white tienilic acid, m.p. 150-153°C., are obtained.

Example 2

10 g of 2,3-dichloro-4-(2-thienylcarbonyl)-phenol, 4.0 g of chloroacetic acid anhydride, 50 ml of toluene, 4.0 ml of dimethylsulfoxide, 0.5 g of Aliquat 336 and 8.0 g sodium carbonate are refluxed for 2 hours. The reaction is then continued as described in Example 1. The yield is 11.5 g (95.0 per cent) of white tienilic acid, m.p. 150.5-153°C.

Example 3

10 g of 2,3-dichloro-4-(2-thienylcarbonyl)-phenol, 4.0 g of chloroacetic acid anhydride, 40 ml of acetone and 5.0 g of potassium carbonate are refluxed for 24 hours. 50 ml of water and 5 ml of 48 per cent sodium hydroxide solution is added and the mixture is refluxed for 2 hours. The solution is cooled, the acetone is distilled off, and the solution is then washed with toluene. The aqueous layer is acidified with hydrochloric acid and the product is extracted with hot toluene. The toluene solution is cooled and the precipitate is filtered off. The yield of white tienilic acid, m.p. 150-153°C., is 8 g (66 per cent).

CLAIMS

1. A process for the preparation of [2,3-dichloro-4-(2-thienylcarbonyl)phenoxy]-acetic acid of the formula:

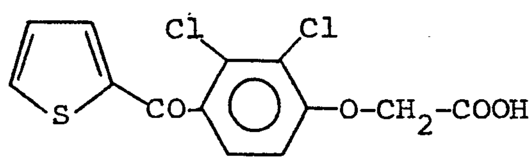

I

which comprises reacting 2,3-dichloro-4-(2-thienylcarbonyl)-phenol with chloroacetic acid anhydride to give [2,3-dichloro-4-(2-thienylcarbonyl) phenoxy]-acetic acid anhydride of the formula:

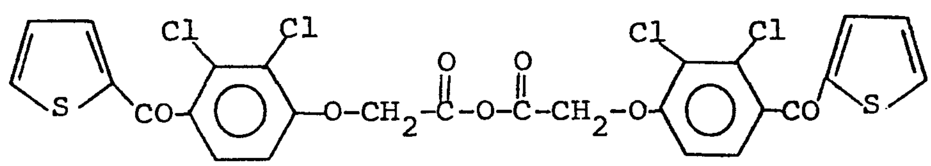

III

and then hydrolysing the latter to give the compound of formula I.

2. A process according to Claim 1, in which the reaction of the said phenol with the chloroacetic acid anhydride is carried out in an aromatic or aliphatic hydrocarbon, ketone or ester, a halogenated hydrocarbon, an organic amide or a sulfoxide.

3. A process according to Claim 1 or 2, in which the said phenol is reacted with chloroacetic acid anhydride in the presence of an alkaline condensation agent.

4. A process according to Claim 3, in which the alkaline condensation agent is sodium carbonate or potassium carbonate.

5. A process according to Claim 3 or 4, in which the said alkaline condensation agent is used in admixture with a quaternary ammonium salt.

6. A process according to Claim 6, in which the said quaternary ammonium salt is trioctylmethylammonium chloride.

7. [2,3-Dichloro-4-(2-thienylcarbonyl)-phenoxy]acetic acid anhydride of the formula:

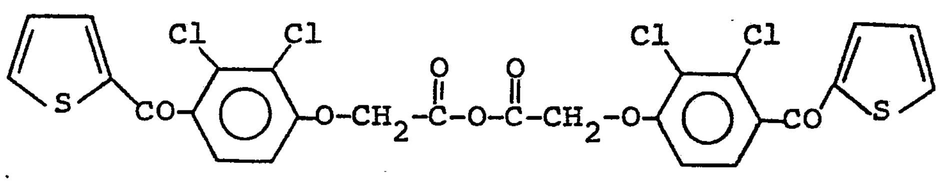

8. Process for the preparation of [2,3-dichloro-4-(2-thienylcarbonyl) phenoxy]acetic acid anhydride which comprises reacting 2,3-dichloro-4-(2-thienylcarbonyl)-phenol with chloroacetic acid anhydride.

9. Process for the preparation of [2,3-dichloro-4-(2-thienylcarbonyl) phenoxy]acetic acid which comprises hydrolysing [2,3-dichloro-4-(2-thienylcarbonyl)phenoxy]acetic acid anhydride.

European Patent Office

# EUROPEAN SEARCH REPORT

Application number

EP 80 30 4227

| DOCUMENTS CONSIDERED TO BE RELEVANT | | | CLASSIFICATION OF THE APPLICATION (Int. Cl.3) |
|---|---|---|---|
| Category | Citation of document with indication, where appropriate, of relevant passages | Relevant to claim | |
| A | US - A - 4 166 061 (A.R. MASTROCOLA)<br>* Claims *<br>-- | 1 | C 07 D 333/22 |
| A | FR - A - 2 364 914 (ALBERT ROLLAND)<br>* Claims *<br>---- | 1 | |
| | | | TECHNICAL FIELDS SEARCHED (Int. Cl.3) |
| | | | C 07 D 333/22 |
| | | | CATEGORY OF CITED DOCUMENTS<br>X: particularly relevant<br>A: technological background<br>O: non-written disclosure<br>P: intermediate document<br>T: theory or principle underlying the invention<br>E: conflicting application<br>D: document cited in the application<br>L: citation for other reasons |
| X | The present search report has been drawn up for all claims | | &: member of the same patent family, corresponding document |

| Place of search | Date of completion of the search | Examiner |
|---|---|---|
| The Hague | 05-02-1981 | CHOULY |